# EUROPEAN PATENT APPLICATION

(11) **EP 4 365 824 A1**
(43) Date of publication of application: **08.05.2024**
(21) Application number: 22205477.7
(22) Date of filing: 04.11.2022
(51) Int. Cl.: G06T 5/00, G06T 9/00, H04N 19/00

(54) **STORING A MEDICAL IMAGE**

(71) Applicant: Koninklijke Philips N.V., 5656 AG Eindhoven (NL)
(72) Inventor: SCHNELLBÄCHER, Nikolas David, Eindhoven (NL); BERGNER, Frank, Eindhoven (NL); GRASS, Michael, 5656AG Eindhoven (NL)
(74) Representative: Philips Intellectual Property & Standards

(57) **Abstract**

Proposed concepts aim to provide schemes, solutions, concepts, designs, methods and systems pertaining to the storage, retrieval and/or communication of medical images. In particular, the medical image is de-noised to generate a (substantially) noise-free medical image. De-noising the medical image to this extent allows for a high compression ratio, and thus space-efficient storage of the medical image. However, typically the level to which medical image is de-noised has to be carefully controlled in order to minimise the chance of introducing errors to the medical image. In the present invention, noise information describing residual noise data removed from the medical image is also stored. This allows for the compressed noise-free medical image to be combined with the noise information at a receiver, restoring any potentially lost information. In this way, a space-efficient means for storing a medical image is provided, which also overcomes problems associated with heavily de-noising the medical image.

## Description

### FIELD OF THE INVENTION

The present invention relates to storing a medical image, and more particularly to compressing and storing a medical image.

### BACKGROUND OF THE INVENTION

Connectivity and data sharing between medical sites is an increasingly important consideration for the seamless operation of medical workflows in a digitized medical data landscape. As a result, it is increasingly standard practice for medical facilities to host their data in cloud and/or web-based systems.

Of particular concern is the storage of medical imaging data on picture archiving and communication systems (PACS). Storage of medical imaging data is inherently data intensive, and therefore presents a bottleneck in many processing chains. While large cloud-hosted databases provide a convenient solution for connectivity between medical sites, they also come with associated disadvantages due to a restricted file-transfer speed between the host and an end-user.

Accordingly, efficient medical image storage concepts are important for reducing the cost of storage, allowing a faster rate of data transfer and processing, and providing an ecological solution on a large scale.

### SUMMARY OF THE INVENTION

The invention is defined by the claims.
Proposed concepts aim to provide schemes, solutions, concepts, designs, methods and systems pertaining to the storage, retrieval and/or communication of medical images. In particular, a medical image is denoised to generate a (substantially) noise-free medical image. De-noising the medical image to this extent allows for a high compression ratio, and thus space-efficient storage of the medical image. However, typically the level to which medical image is de-noised has to be carefully controlled in order to minimise the chance of introducing errors. In the present invention, noise information describing residual noise data removed from the medical image is also stored. This allows for the compressed noise-free medical image to be combined with the noise information at a receiver, restoring any potentially lost information. In this way, a space-efficient means for storing a medical image is provided, which also overcomes problems associated with heavily de-noising the medical image.

According to examples in accordance with an aspect of the invention, there is provided a method of storing a medical image, the method comprising:
processing, with a de-noising algorithm, a medical image to generate a substantially noise-free medical image and associated residual noise data describing noise removed from the medical image;
processing, with an image compression algorithm, the substantially noise-free medical image to generate a compressed medical image; and
storing the compressed medical image and associated noise information describing the residual noise data.

In this way, embodiments of the invention may be able to store (i.e. in a local memory, a cloud memory, etc.) a medical image with a high compression ratio, while not sacrificing image integrity. Typically, medical images are carefully denoised in order to not distort the image in any way. This means that a potential compression ratio is decreased, as an image with a lower amount of noise may be compressed to a greater extent.

However, the present invention de-noises the medical image to create a (substantially) noise-free medical image, thus providing an image that may be compressed to a greater extent. Information regarding the noise removed/deleted/extracted from the medical image is also stored. As a result, when the image is decompressed, noise may also be added back to the de-compressed medical image to restore the medical image to its original state. Thus, the invention overcomes issues associated with de-noising medical images to an extent that the medical image is (substantially) noise-free. Consequently, this allows the compression of the medical image to a greater extent, reducing an amount of memory required to store the medical image.

By way of explanation, usual de-noising algorithms for medical images are adapted to allow de-noising up to a point where de-noising errors do not affect clinical decisions. Indeed, when denoising is performed on a medical image, certain image details may be erroneously interpreted as noise by the de-noising algorithm (i.e. due to the imperfection of medical images). Put another way, noise estimations taken by de-noising algorithms are often imperfect. Furthermore, there exist legal obligations not to alter medical images to an extent that structures are shown that may/may not be real (i.e. there exists a degree of uncertainty of an object/structure actually being present, and not an imaging error or image reconstruction artifact).

Thus, the point where the de-noising algorithm begins to reach conclusions with low levels of certainty needs to be carefully assessed during the tailoring of the de-noising algorithm. This inevitably leads to the presence of residual noise in the medical image even after de-noising has been performed. Accordingly, a compression ratio of the resultant de-noised medical image may be reduced.

Embodiments of the invention are therefore based on the realization that, by storing residual noise data describing noise removed from the medical image, the above problem may be suppressed. The medical image can be de-noised to an extent that would not typically happen (due to the risk of introducing errors), as the residual noise data can be used during reconstruction to produce a faithful reconstruction of the medical image. Thus, a medical image can be obtained from storage with noise artificially restored. Accordingly, a higher compression ratio may be achieved using existing compression algorithms.

Put another way, the medical image is de-noised to an extent that a substantially noise-free medical image is obtained. A subsequent compression step in the image-domain benefits from the noise-free nature of the medical image, allowing high compression ratios (even for lossless schemes). For subsequent medical image retrieval and viewing the required amount of residual noise can be adaptively mixed back into the medical image. This may be achieved by explicitly storing the noise removed from the image, or by virtue of a generative noise model which reproduces the image noise characteristics based on stored residual noise information (e.g. statistical moments, correlation functions or noise power spectrum).

In some embodiments, the de-noising algorithm may remove noise from the medical image to an extent that de-noising errors are present in the substantially noise-free medical image.

Current techniques do not allow for the de-noising of a medical image to a point that denoising errors may occur. For example, a (poorly imaged) structure may actually be present, but may be interpreted as noise by a de-noising algorithm and removed accordingly. Alternatively, a structure that is not actually present in the image (but seemingly present due to noisy imaging) may be interpreted as present by the de-noising algorithm. By removal of the noise, said fake structure may be emphasized, and therefore artificially inserted into the image.

The above limit to de-noising inherently means that a compression ratio of the resultant de-noised medical image is limited. Embodiments of the invention allow for the de-noising of medical images beyond the above limit, as information of the noise is stored, which may then be restored when retrieving the medical image. Accordingly, higher compression ratios are enabled, whilst avoiding problematic errors introduced to the ultimately presented/viewed medical image.

In some embodiments, the de-noising algorithm may be a machine learning based denoising algorithm. In some cases, the de-noising algorithm may be a deep convolutional neural network based de-noising algorithm.

Machine learning based de-noising algorithms present a powerful denoising tool, in that they may be more effective (i.e. efficient, accurate, etc.) at identifying and removing noise from an image. However, such algorithms are often not appropriate for de-noising medical images, as they may introduce errors in the de-noising that may negatively impact clinical decisions. Indeed, machine learning based denoising algorithms are liable to show images that are too perfect (i.e. with potential structures removed), and/or show overly realistic structures (i.e. introduce non-existent structures).

Accordingly, the invention enables the use of such de-noising algorithms, as any potential errors introduced by de-noising will be negated by the re-introduction of noise after decompression.

In further embodiments, the noise information may comprise at least a part of the residual noise data.

The actual noise removed (i.e. the intensity and position of noise-related pixels) may be stored, at least in part. This enables completely accurate reconstruction of the noise when received from storage.

In particular embodiments, the method may further comprise processing, using a data compression algorithm, at least a part of the residual noise data to generate compressed noise data. In this case, the noise information may comprise the compressed noise data.

While storing (part) of the actual noise may enable perfect/faithful reconstruction of the medical image, this may prove to be a large amount of data. Thus, by compressing this residual noise data, an efficiency of storage may be improved.

In some embodiments, the method may also comprise analyzing the residual noise data to generate the noise information. In this case, the noise information may comprise noise parameters including at least one of: a noise model type, a noise magnitude, a noise power spectrum, a modulation transfer function, and medical image acquisition parameters.

Alternatively (or in addition to) to directly storing the noise, information describing the residual noise data may be generated and stored. It is often the case that noise follows a probabilistic distribution, which may be characterized by a handful for parameters. Accordingly, noise can be reintroduced to the medical image by a receiver that can generate the noise based on the noise information. This characterization of the noise may not require as much storage space as storing the residual noise itself, thus saving storage space and reducing file transfer time.

In some embodiments, the image compression algorithm may be one of a wavelet based compression algorithm, or a neural network based compression algorithm.

Certain compression algorithms may benefit from the high level of de-noising to provide higher compression ratios than other algorithms. Two such algorithms may be wavelet-based and neural network based compression algorithms.

In some embodiments, the compressed medical image and the noise information are stored in a cloud storage.

Embodiments of the present invention may be particularly beneficial for cloud storage applications. Cloud storage experiences bottlenecks when it comes to downloading/uploading to the cloud, particularly for large file sizes. By the present invention, higher compression ratios, and thus smaller file sizes, may be achieved. Thus, embodiments may enable cloud storage to be a more effective means of storing medical images.

According to other examples in accordance with an aspect of the invention, there is provided a method of retrieving a medical image from storage, comprising:
obtaining, from storage, a compressed medical image and associated noise information describing residual noise data, wherein the residual noise data describes noise removed from a medical image corresponding to the compressed medical image;
processing, using an image decompression algorithm, the compressed medical image to generate a substantially noise-free medical image; and
generating the medical image based on the substantially noise-free medical image and the noise information.

Obtaining stored data in this way enables the faithful reconstruction of a medical image. While simply decompressing a medical image provides a medical image viewable for diagnosis/analysis, such images may have errors introduced by a de-noising prior to compression during storage. Thus, by also retrieving noise information describing residual noise data, a medical image that more accurately represents the medical image initially captured by a scanner/instrument can be obtained.

Put another way, reintroducing noise into the medical image may reduce the occurrence of errors that may mislead a caregiver observing such an image. Indeed, de-noised medical image may be missing structures present in the original image, or may erroneously contain structures that do not actually exist. Mixing the de-compressed medical image with the residual noise data can lead to more accurate conclusions regarding the medical image.

As the residual noise is reintroduced, the medical image may be initially de-noised to a point where the medical image is (substantially) noise free. This facilitates higher compression ratios for the medical image. In this way, an amount of storage required for the medical image is decreased, and a download/upload time to storage may also be decreased.

In some related embodiments, generating the medical image may comprise: analyzing the noise information to determine the residual noise data associated with the medical image; and generating the medical image based on the substantially noise-free medical image and the residual noise data.

The medical image is produced based on the (substantially) noise-free medical image and residual noise data extracted from the noise information. Analyzing the noise information may comprise receiving residual noise power spectral information, mode and statistical moments of an underlying (noise) distribution to interpret the nature of the noise removed from the medical image.

In some embodiments, generating the medical image may comprise merging the substantially noise-free medical image and at least a part of the residual noise data.

In further embodiments, generating the medical image further comprises obtaining user preferences describing a target noise level of the medical image. In this case, generating the medical image may be further based on the user preferences.

As a result, a user (i.e. a caregiver/medical professional) can selectively choose the amount of noise reintroduced to the medical image. The user may be highly trained, and therefore be able to more accurately assess whether the noise is useful for diagnostic purposes (i.e. is simply noise, or has a chance of corresponding to poorly imaged structures). Thus, this allows the user to obtain a medical image that more accurately represents a ground truth of the imaged subject.

According to further examples in accordance with an aspect of the invention there is provided a method of communicating a medical image, comprising storing the medical image and retrieving the medical image as described above.

According to yet further examples in accordance with an aspect of the invention, there is provided a computer program comprising computer program code means adapted, when said computer program is run on a computer, to implement a method of storing, retrieving or communicating a medical image.

According to additional examples in accordance with an aspect of the invention, there is provided a system for storing a medical image, the system comprising:
a de-noiser configured to process, with a de-noising algorithm, a substantially noise-free medical image and associated residual noise data describing noise removed from the medical image;
a compressor configured to process, with an image compression algorithm, the substantially noise-free medical image to generate a compressed medical image; and
an interface configured to store the compressed medical image and associated noise information describing the residual noise data.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a graph representing a relationship between de-noising of an image and an achievable compression ratio;
Fig. 2 presents a flow diagram of a method for storing a medical image according to an embodiment of the invention;
Fig. 3 presents a flow diagram of a method for retrieving a medical image according to another embodiment of the invention;
Fig. 4 is a simplified block diagram including a system for storing a medical image according to a further embodiment of the invention, and a system for retrieving the medical image according to another embodiment of the invention;
Fig. 5 is a simplified block diagram of a system for communicating medical images; and
Fig. 6 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should also be understood that the Figures are merely schematic and are not drawn to scale. It should also be understood that the same reference numerals are used throughout the Figures to indicate the same or similar parts.

The invention proposes concepts for storage, retrieval and/or communication of medical images. In particular, the medical image is de-noised to generate a (substantially) noise-free medical image. De-noising the medical image to this extent allows for a high compression ratio, and thus space-efficient storage of the medical image. However, typically the level to which a medical image is denoised has to be carefully controlled in order to minimise the chance of introducing errors or depends on the clinical protocol that is used by a clinical expert. In the present invention, noise information describing residual noise data removed from the medical image is also stored. This allows for the compressed noise-free medical image to be combined with the noise information at a receiver, restoring any potentially lost information. In this way, a space-efficient means for storing a medical image is provided, which also overcomes problems associated with heavily de-noising the medical image.

Put another way, embodiments of the invention overcome problems associated with an increasing data demand in medical workflows, and associated large file sizes involved in such workflows. To this effect, an effective (i.e. efficient) de-noising and compression scheme is provided by embodiments, which allows high compressions ratios whilst ensuring good medical image quality (i.e. a suppression of errors introduced by de-noising), so as to maintain a high diagnostic image quality. Since cloud-hosted databases can become cost-expensive and a processing bottleneck due to file-transfer speed limitations between host and end-user, embodiments address two important concerns for professional medical facilities.

Proposed medical image communication systems may therefore include a de-noising module for medical image-based noise removal, a compression module for the medical image and/or noise information compression, and a noise insertion module for recreation of a desired amount of noise within the medical image for viewing/medical reading.

It should be appreciated that proposed concepts are suitable for many medical image forms/modalities. By way of non-restrictive example, CT, MR, ultrasound, PET, SPECT, OCT and IVUS medical images may benefit from embodiments of the proposed invention. However, the skilled person would readily appreciate further medical-related images that may benefit from a de-noising and compression scheme that minimises the chance of introducing errors that may influence clinical decisions.

For the avoidance of confusion, the rational for adding back noise into the medical image is that typical de-noising algorithms are tailored/adapted/configured to only allow de-noising up to a point where de-noising errors do not affect clinical decisions. Noise estimations performed by de-noising algorithms are always liable to imperfections (i.e. it is impractical to extract/correct every noise-related pixel from an image), and in the realm of medical technologies there exist obligations (legal and moral) to not show structures that might not be real. Such structures may erroneously/incidentally be included due to de-noising algorithms or other processing steps, or structures may actually be interpreted as noise and removed. In particular, AI/machine learning based de-noising algorithms may be more liable to errors, such as showing images that are too perfect, or extrapolate fake structures such that they are portrayed as realistic. The point where guessing becomes a factor needs to be carefully assessed during the tailoring of the de-noising algorithms for this purpose. This ends up imposing a minimum amount of noise that is likely to be present in the medical image.

Thus, embodiments of the invention provide the (at least partial) storage of the residual noise removed from the image, and/or information characterizing the noise. The removed/residual noise may then be artificially restored when retrieved from storage, thus recreating a faithful representation of the original medical image with the original (or adapted) level of noise.

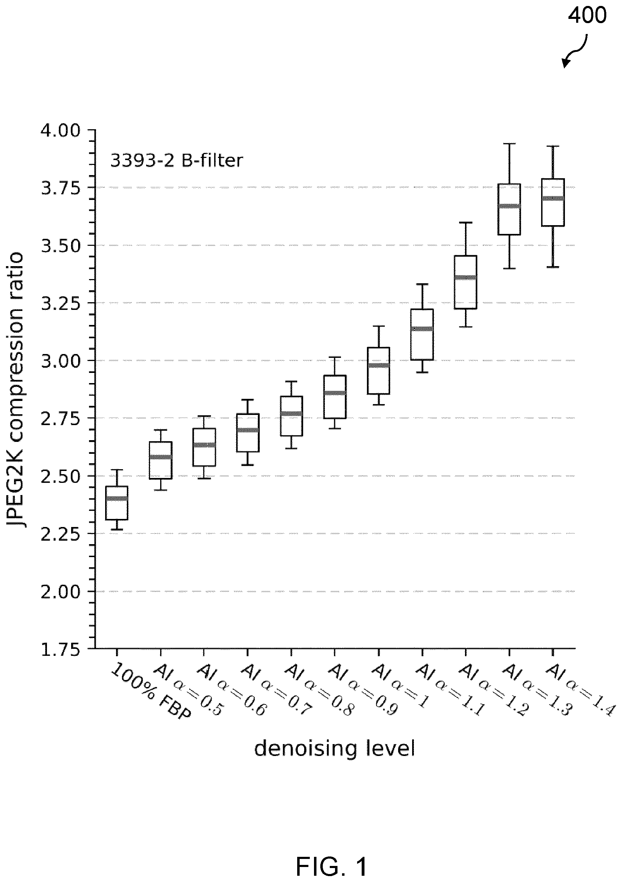

As a result of enabling an increased level of de-noising of the medical image to a (substantially) noise-free level, a higher compression ratio of the medical image may be achieved (compared to de-noising the medical image to a lesser extent). This is demonstrated in Fig. 1, which is a graph representing a relationship between de-noising of an image and an achievable compression ratio.

Specifically, Fig. 1 shows example results for a compression of a CT image where a lossless wavelet-based JPEG-2000 compression is applied to conventional CT medical images subject to de-noising algorithms of differing strength. An increase on the x-axis indicates de-noising of a medical image to a higher degree, while an increase on the y-axis indicates a greater compression ration. Thus, the graph clearly shows that there exists higher achievable compression ratios for medical images that have had subject to a stronger de-noising scheme.

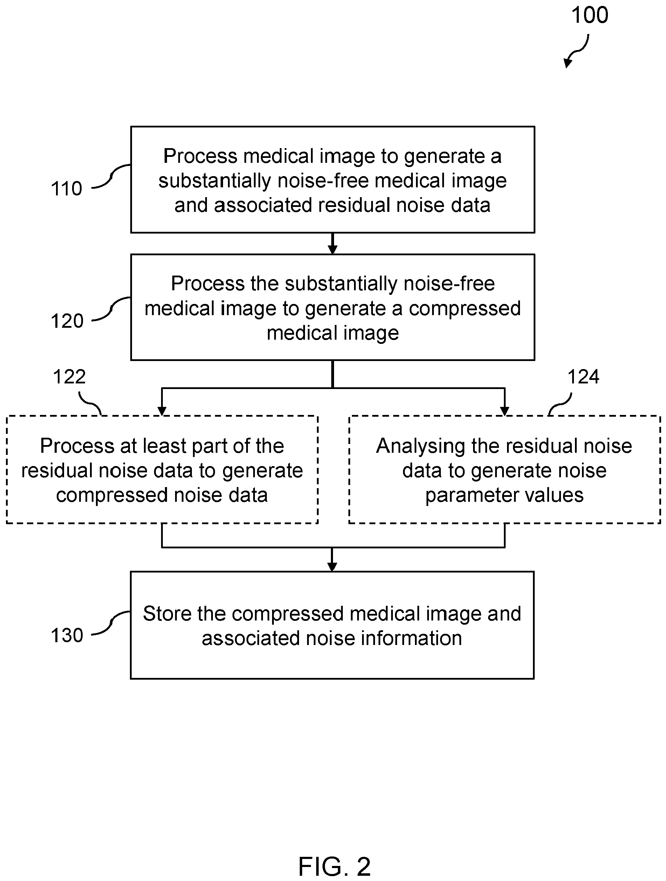

Turning now to Fig. 2, there is presented a flow diagram of a method for storing a medical image according to an embodiment of the invention. Storing the medical image may involve transmitting the medical image for remote storage, or storage may be performed on local memory. The medical image may be any image that is related to the imaging of a subject/subjects. For example, the medical image may be a CT image, an MR image, etc.

At step 110, the medical image is processed with a de-noising algorithm. The de-noising algorithm generates a substantially noise-free medical image and associated residual noise data describing noise removed from the medical image.

Put another way, a de-noising algorithm/method is employed to remove noise from the medical image such that (substantially) all noise from the medical image is removed. The removed noise is represented by the residual noise data.

The substantially noise-free medical image corresponds to the medical image but with irregularities that do not correspond to actual features intended to be captured by the medical image removed. Of course, preferably the noise-free medical image is completely noise free. However, it is often the case that differentiating between noise and features of a medical image is highly difficult, if not impossible. Therefore, noise of the medical image is removed as far as it can be effectively, and confidently identified by the de-noising algorithm.

In some embodiments, the de-noising algorithm removes noise from the medical image to an extent that de-noising errors are present in the substantially noise-free medical image. Indeed, any denoising algorithm performing de-noising on a medical image will introduce errors if performing a strong level of de-noising. Such errors may be in the form of erroneous removal of data, or erroneous correction of pixels. This may mean that certain structures are removed, or over emphasised in the de-noised medical image. Of course, this may influence clinical/diagnostic decisions, but noise may be reintroduced due to the storage of associated noise information, effectively combating this issue. It should be noted that, while de-noising to this extent while saving the noise information may be counter-intuitive, denoising to this extent allows for a higher compression ratio of the medical image.

Furthermore, the residual noise data describes the noise (i.e. random fluctuations during medical image acquisition) that is removed from the medical image by the de-noising algorithm. Thinking from another perspective, a combination of the substantially noise-free medical image and the residual noise data would enable the restoration of the original medical image.

The de-noising algorithm may be any de-noising algorithm/method suitable for removing noise (i.e. random fluctuations during medical image acquisition) from a medical image. Such algorithms would be readily appreciated by the skilled person. For example, the de-noising algorithm may be a classic de-noising algorithm such as a total variation, bilateral filter, non-local means, BM3D or Bregmann iteration algorithm.

In exemplary embodiments, the de-noising algorithm is a machine learning (AI/neural network) based de-noising algorithm. Such algorithms may be able to identify and correct noise-related pixels more effectively than classic de-noising algorithms. In specific cases, the de-noising algorithm may be a deep convolutional neural network based de-noising algorithm.

At step 120, the substantially noise-free medical image is processed with an image compression algorithm to generate a compressed medical image.

The compression of the substantially noise-free medical image benefits from the (substantially) noise-free nature of the image, such that high compression ratios may be achieved by known image compression algorithms.

The compression algorithm may be lossy or lossless, depending on the importance of veracity of the medical image to be compressed. For example, the image compression algorithm may be a wavelet-based lossless JPEG2000 compression algorithm, or a neural network based compression algorithm (e.g. a variational auto-encoder). Of course, the skilled person would readily appreciate other image compression algorithms suitable for this purpose.

At step 130, the compressed medical image and associated noise information describing the residual noise data are stored.

The compressed medical image and associated noise information may advantageously be stored in a cloud storage, such that they may be acquired by any entity with adequate permissions to access such information. Thus, file-sharing and transfer is facilitated. Alternatively, the compressed medical image and associated noise information may be stored locally, such as on a local network or on local (i.e. device specific) storage.

By way of brief explanation, the noise information may comprise (in some embodiments) at least a part of the residual noise data. In this way, (a part of) the residual noise data (being noise data removed from the medical image) is directly stored. For example, this may correspond to the exact alterations to the medical image performed to acquire the substantially noise-free medical image, such as a list of pixel coordinates and intensity value changes.

In some embodiments, the method may further comprise one of optional steps 122 and 124, or both optional steps 122 and 124.

In step 122, at least a part of the residual noise data is processed using a data compression algorithm in order to generate compressed noise data. In this case, the noise information comprises the compressed noise data. The data compression algorithm may be similar to the image compression algorithm outlined above, or may be a different compression algorithm. In any case, the compressed noise data has a smaller storage footprint than the residual noise data, ensuring more efficient storage.

In step 124, the residual noise data is analyzed to generate the noise information. The noise information in this case comprises noise parameter values including at least one of: a noise model type, a noise magnitude, a noise power spectrum, a modulation transfer function, and medical image acquisition parameters.

The noise parameter values therefore characterize the residual noise data in an abstract way, which may be used for subsequent regeneration of the noise (rather than simply reintroducing the exact noise). Indeed, the residual noise data will often follow a probabilistic distribution, which may be summarized by the noise parameter values (e.g. statistical moments or correlation functions).

To paraphrase the above, the noise information describes/is representative of the residual noise data removed from the medical image to generate the substantially noise-free medical image. The noise information comprises at least one of: a part/all of the residual noise data, a compressed part/all of the residual noise data, and noise parameter values characterizing the residual noise data. The noise information may thus be used to reintroduce/mix noise back into a decompressed medical image subsequent to storage, restoring the original medical image.

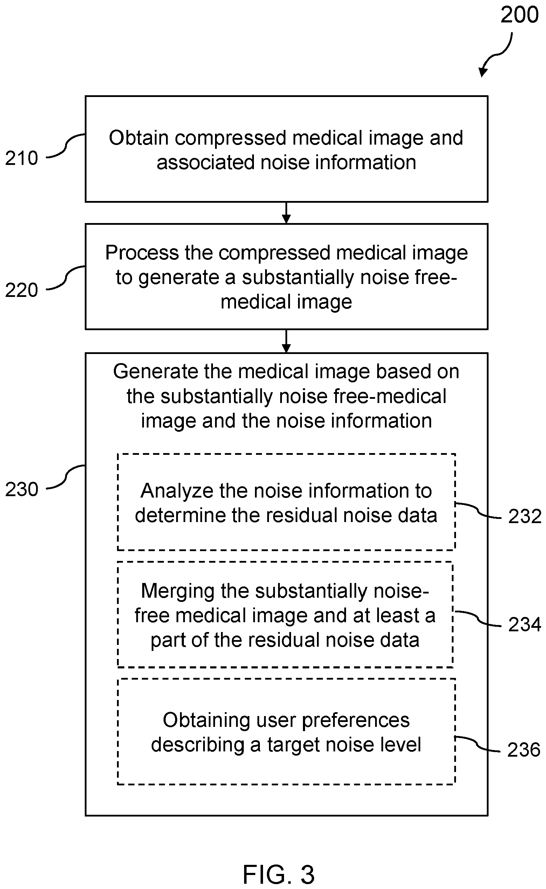

Fig. 3 presents a flow diagram of a method for retrieving a medical image from storage according to another embodiment of the invention. The medical image may be stored according to a method described in relation to Fig. 2, or may be stored in another manner.

Firstly, at step 210, a compressed medical image and associated noise information describing residual noise data is obtained from storage. The residual noise data describes noise removed from a medical image corresponding to the compressed medical image.

In other words, a compressed medical image is retrieved from storage (i.e. local storage, from the cloud, etc.). The compressed medical image is a medical image that was initially de-noised to an extent that it was a substantially noise-free medical image, and subsequently compressed. Accordingly, the compressed medical image may be a highly compressed medical image (i.e. having a high compression ratio).

Along with the compressed medical image, noise information is also obtained/retrieved. The noise information may be obtained from the same place/location as the compressed medical image, or may be obtained from a separate storage. The noise information describes residual noise data, which in turn describes noise removed from the medical image to produce a substantially noise-free medical image. Thus, the noise information represents information removed from the medical image prior to compression of the medical image.

At step 220, the compressed medical image is processed using a decompression algorithm to generate a substantially noise-free medical image. This may be achieved in any manner known in the art of image decompression algorithms. Essentially, this decompresses the medical image into a state that it was before compression for storage.

At step 230, the medical image is generated based on the substantially noise-free medical image and the noise information. In other words, the noise information is used to insert/mix/combine noise with the medial image, to reproduce a medical image with noise embedded within it.

As a result, a medical image is reproduced, reducing the number of possible errors introduced by a de-noising algorithm applied to the original medical image. This may ensure that the method of storing and retrieving the medical image does not have a large negative impact on clinical/diagnostic decisions.

In other words, at step 230 a medical image is restored to a state in which it is not denoised to be (substantially) noise-free. This means that noise is reintroduced into the image. While this may seem counter-intuitive, de-noising algorithms are liable to removing information useful for diagnostic purposes/adding information that may be deceiving. Thus, by reintroducing (at least a part of) the noise, an accurate representation of the original medical image may be acquired and beneficial for diagnostic assessment.

At (optional) sub-step 232, the noise information is analyzed to determine the residual noise data associated with the medical image. Indeed, this may include extracting noise parameter values including at least one of: a noise model type, a noise magnitude, a noise power spectrum, a modulation transfer function, and medical image acquisition parameters. This information describes a noise originally removed from the medical image. Then, the medical image is generated based on the substantially noise-free medical image and the residual noise data.

At (optional) sub-step 234, the medical image comprises merging the substantially noise-free medical image and at least a part of the residual noise data.

At (optional) sub-step 236, user preferences are obtained, which describe a target noise level of the medical image. In this case, generating the medical image is further based on the user preferences.

Moreover, it should be noted that the embodiments of the methods described in reference to Fig. 2, and embodiments of methods described in reference to Fig. 3, may be selectively combined to provide a method of communicating a medical image (via storage).

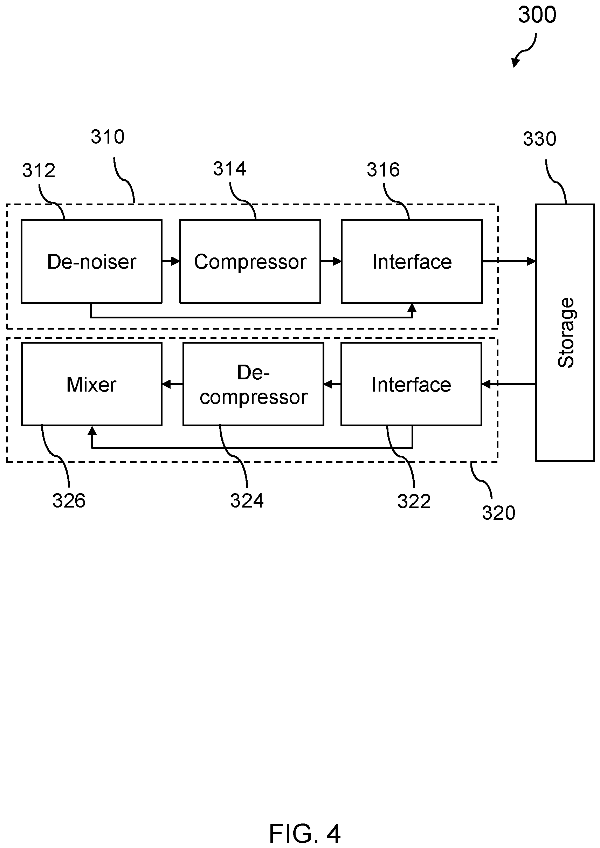

Fig. 4 is a simplified block diagram 300 including a system 310 for storing a medical image according to a further embodiment of the invention, and a system 320 for retrieving the medical image according to another embodiment of the invention.

The system 310 for storing the medical image comprises a de-nosier 312, a compressor 314 and an interface 316.

The de-noiser 312 is configured to process, with a de-noising algorithm, a substantially noise-free medical image and associated residual noise data describing noise removed from the medical image.

The compressor 314 is configured to process, with an image compression algorithm, the substantially noise-free medical image to generate a compressed medical image.

The interface 316 is configured to store the compressed medical image and associated noise information describing the residual noise data.

The system 320 for retrieving the medical image comprises an interface 322, a de-compressor 324, and a mixer 326.

The interface 322 is configured to obtain, from storage 330, the compressed medical image and the associated noise information.

The de-compressor 324 is configured to process, using an image decompression algorithm, the compressed medical image to generate the substantially noise-free medical image.

The mixer 326 is configured to generate the medical image based on the substantially noise-free medical image and the noise information.

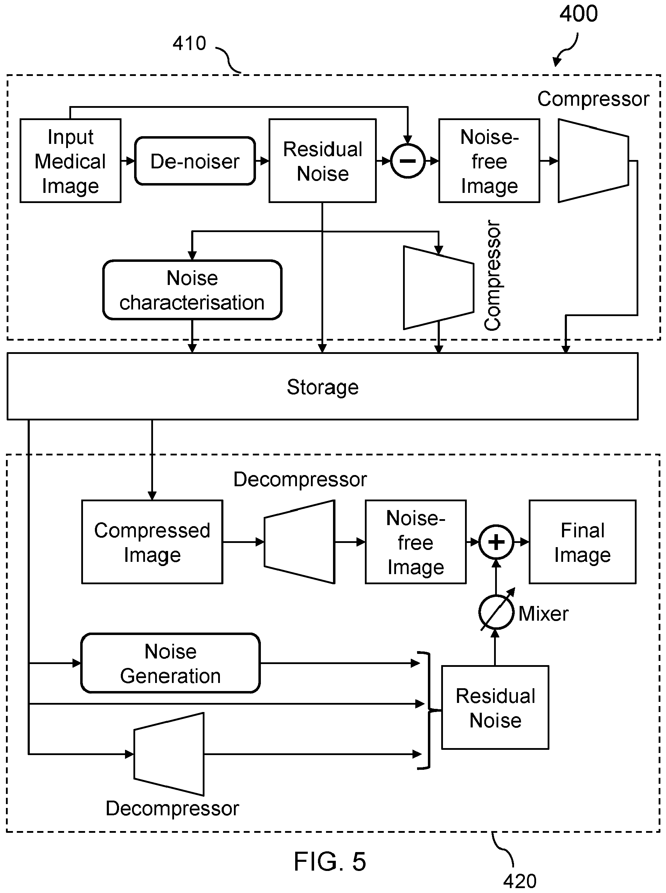

Fig. 5 is a schematic diagram of a system 400 for communicating medical images, including a forward pass 410 for joint compression and de-noising of a medical image, and a backward pass 420 for decompression and noise (re-)generation of the medical image, according to a specific embodiment.

The de-noiser consists of a denoising algorithm tailored to the medical imaging modality (i.e. CT, MR image etc.) or clinical protocol at hand. The denoising algorithm can be a classical denoising algorithm (e.g. total variation (TV), bilateral filter, non-local means (NLM), BM3D, Bregmann iteration, etc.). Alternatively, the denoising algorithm can be machine learning-based algorithm, such as a deep CNN based denoising model.

The generation of the input image (i.e. the noise-free medical image) for the compression stage is then best realized by using a strong de-noising setting to produce flat substantially/virtually noise-free medical images up to the extent which does not hamper with diagnostic quality while preserving anatomical detail. This can either be realized by a residual scaling technique, in which over-correction of the predicted noise residuum is performed in order to arrive at virtually noise-free images. Alternatively, or additionally, an iterative solution may be performed for de-noising, which is stopped when a (sufficiently) noise-free texture of the medical image is achieved.

By way of specific example, for CT applications it may be beneficial to apply the image-based de-noising prior to applying the reconstruction kernel, which produces the final image characteristics (image sharpness, NPS, MTF). This removes the filter-dependency for the compression and noise insertion step, and thus allows the faithful recovery of noise characteristics by applying the analytically known transfer function to the final image as the last processing step after the noise insertion has already been performed.

The compressor module consists of a compression algorithm, which benefits from the reduced noise characteristics of the provided substantially noise-free medical image in terms of higher achievable compression rates, the less noisy the input image is. This compression algorithm can be a lossless or lossy compression method of choice.

The compression may be, for example, a lossless JPEG2000 compression algorithm (wavelet-based). It could also be other lossless or lossy compression schemes, including neural-network compression algorithms, such as variational auto-encoders (VAEs). If the residual noise is directly stored, the compression scheme may also be extended to the residual noise image.

The noise capture module may be provided in the forward pass 410 in order to capture the removed noise from the medical image, such that it can be restored using an adaptive noise-level for noise insertion in the backward 420 pass for original medical image retrieval, and at the same time allow for data-efficient storage along-side the compressed image.

As a first embodiment, noise characterization may be performed which captures the noise characteristics of the residual noise (i.e. the noise removed from the medical image) in terms of a set of noise parameters, which can be stored in the (compressed) image volume, e.g. in DICOM tags as metadata. The noise parameters may comprise:
(i) a noise-model name (e.g. Gaussian, Riccian, Poisson, Salt-and-pepper, shot-noise, count-noise or more complicated handcrafted noise models) to denote an underlying noise model. This can also be a convolution of several noise sources which are convolved to a single noise model;
(ii) a (scalar) magnitude parameter capturing the noise level;
(iii) NPS (noise power spectrum) and MTF (modulation transfer function) measurements, describing the noise characteristics, and important for preserving realistic image textures; and/or
(iv) a scan geometry module to take the dependency of the noise pattern w.r.t. scan /acquisition parameters into account.

Alternatively (or additionally), the removed noise is directly stored as a full residuum.

Alternatively (or additionally), the residual noise may undergo a similar compression scheme as does the image itself. Here the employed compression algorithm may be (but equally may not be) identical to the image-based compressor.

In some embodiments, the mixer is adaptable to user preferences and results may vary depending on the user retrieving the data from storage/the cloud. Put another way, noise may be mixed with the noise-free medical image based on user preferences.

The noise generation module will create the requested noise for the three described embodiments above.

The noise generator can also employ a generative data-driven model which can produce faithful noise images based on the meaningful set of noise-parameters outlined above, which may have been stored in the meta-file header of the compressed noise-free medical image. This information can also encompass physical scan and acquisition parameters which allow a fine tuning of the mixer to correctly model the underlying physical noise sources.

Finally, the backward 420 pass may also include a clinician in the loop who can request a medical image volume for viewing and actively specifying a desired target noise level. This can be either manually requested by the user at the viewing workstation or set by consolidated default pre-set options for specific clinical protocols.

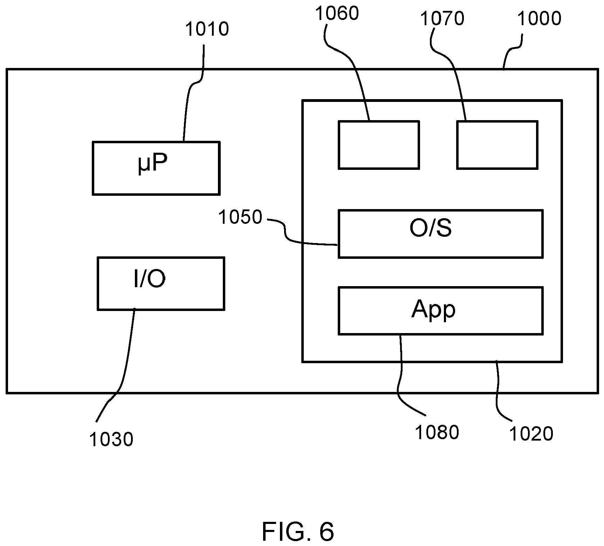

Fig. 6 illustrates an example of a computer 1000 within which one or more parts of an embodiment may be employed. Various operations discussed above may utilize the capabilities of the computer 1000. For example, one or more parts of a system for storing, retrieving and/or communicating a medical image may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of readonly-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figs. 2 and 3, and the systems described in relation to Figs. 4 and 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram Figs. 4 and 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) of storing a medical image, the method comprising:
processing (110), with a de-noising algorithm, a medical image to generate a substantially noise-free medical image and associated residual noise data describing noise removed from the medical image;
processing (120), with an image compression algorithm, the substantially noise-free medical image to generate a compressed medical image; and
storing (130) the compressed medical image and associated noise information describing the residual noise data.

2. The method of claim 1, wherein the de-noising algorithm removes noise from the medical image to an extent that de-noising errors are present in the substantially noise-free medical image.

3. The method of claim 1 or 2, wherein the de-noising algorithm is a machine learning based de-noising algorithm, and is optionally a deep convolutional neural network based de-noising algorithm.

4. The method of any of claims 1-3, wherein the noise information comprises at least a part of the residual noise data.

5. The method of any of claims 1-4, further comprising processing (122), using a data compression algorithm, at least a part of the residual noise data to generate compressed noise data, wherein the noise information comprises the compressed noise data.

6. The method of any of claims 1-5, further comprising analyzing (124) the residual noise data to generate the noise information, and
wherein the noise information comprises noise parameter values including at least one of: a noise model type, a noise magnitude, a noise power spectrum, a modulation transfer function, and medical image acquisition parameters.

7. The method of any of claims 1-5, wherein the image compression algorithm is one of a wavelet based compression algorithm, or a neural network based compression algorithm.

8. The method of any of claims 1-6, wherein the compressed medical image and the noise information are stored in a cloud storage.

9. A method (200) of retrieving a medical image from storage, comprising:
obtaining (210), from storage, a compressed medical image and associated noise information describing residual noise data, wherein the residual noise data describes noise removed from a medical image corresponding to the compressed medical image;
processing (220), using an image decompression algorithm, the compressed medical image to generate a substantially noise-free medical image; and
generating (230) the medical image based on the substantially noise-free medical image and the noise information.

10. The method of claim 9, wherein generating the medical image comprises:
analyzing (232) the noise information to determine the residual noise data associated with the medical image;
generating (230) the medical image based on the substantially noise-free medical image and the residual noise data.

11. The method of claim 10, wherein generating the medical image comprises merging (234) the substantially noise-free medical image and at least a part of the residual noise data.

12. The method of any of claims 9-11, wherein generating the medical image further comprises:
obtaining (236) user preferences describing a target noise level of the medical image, wherein generating the medical image is further based on the user preferences.

13. A method of communicating a medical image, comprising:
storing (100) the medical image according to any of claims 1-8; and
retrieving (200) the medical image according to any of claims 9-12.

14. A computer program comprising computer program code means adapted, when said computer program is run on a computer, to implement the method of any of claims 1-13.

15. A system (310) for storing a medical image, the system comprising:
a de-noiser (312) configured to process, with a de-noising algorithm, a substantially noise-free medical image and associated residual noise data describing noise removed from the medical image;
a compressor (314) configured to process, with an image compression algorithm, the substantially noise-free medical image to generate a compressed medical image; and
an interface (306) configured to store the compressed medical image and associated noise information describing the residual noise data.
